# EUROPÄISCHE PATENTSCHRIFT

(11) **EP 1 487 496 B1**
(45) Veröffentlichungstag und Bekanntmachung des Hinweises auf die Patenterteilung: **24.05.2006**
(21) Anmeldenummer: 03712056.5
(22) Anmeldetag: 19.03.2003
(51) Int. Cl.: A61K 47/14, A61K 47/22, G01N 33/15, G01N 13/00

(54) **TESTSYSTEM ZUR EVALUIERUNG DER KOMPATIBILITÄT BIOLOGISCH AKTIVER SUBSTANZEN MIT COPOLYMEREN**
TEST SYSTEM FOR EVALUATING THE COMPATIBILITY OF BIOLOGICALLY ACTIVE SUBSTANCES WITH COPOLYMERS
SYSTEME D'ESSAI PERMETTANT D'EVALUER LA COMPATIBILITE DE SUBSTANCES BIOLOGIQUEMENT ACTIVES AVEC DES COPOLYMERES

(30) Priorität: 25.03.2002 DE 10213242
(43) Veröffentlichungstag der Anmeldung: 22.12.2004
(73) Patentinhaber: Abbott GmbH & Co. KG, 65205 Wiesbaden (DE)
(72) Erfinder: BREITENBACH, Jörg, 68199 Mannheim (DE); LIEPOLD, Bernd, 69121 Heidelberg (DE)
(74) Vertreter: Reitstötter - Kinzebach
(86) Internationale Anmeldenummer: PCT/EP2003/002872
(87) Internationale Veröffentlichungsnummer: WO 2003/080120

(56) Entgegenhaltungen:
- EP-A- 0 987 549
- DE-A- 19 641 437
- US-A- 3 389 174

## Beschreibung

Die vorliegende Erfindung betrifft ein als Testlösungsmittel dienendes flüssiges Gemisch sowie ein Testsystem und ein Verfahren zur Evaluierung der Kompatibilität biologisch aktiver Substanzen mit N-Vinylpyrrolidon-Copolymeren unter Verwendung des flüssigen Gemischs.

Feststoffdispersionen, d.h. homogene feinstdisperse Phasen von zwei oder mehreren Feststoffen und der Sonderfall der sogenannten festen Lösungen (molekulardisperse Systeme), sowie ihr Einsatz in der pharmazeutischen Technologie sind allgemein bekannt, vgl.Chiou und Riegelman J. Pharm. Sci., 60, 1281 - 1300 (1997).

Die Herstellung von festen Lösungen kann mit Hilfe von Schmelzeverfahren oder nach dem Lösungsverfahren erfolgen. Als polymerer Hilfsstoff für die Herstellung solcher Feststoffdispersionen bzw. fester Lösungen eignen sich insbesondere N-Vinylpyrrolidon-Copolymere, d.h. Copolymere des N-Vinylpyrrolidons mit weiteren ethylenisch ungesättigten Monomeren. Besonders vorteilhaft lassen sich feste Lösungen von biologisch aktiven Substanzen auf Basis derartiger Copolymere durch Schmelzextrusion herstellen, wie beispielsweise in der EP-A 240 904 beschrieben ist.

Die Herstellung von Schmelzextrudaten stellt jedoch Mindestanforderungen an die eingesetzten Mengen. Stehen nur kleinere Wirkstoffmengen zur Verfügung, so lässt sich nicht mit Sicherheit vorhersagen, ob ein Wirkstoff zusammen mit dem gewählten Copolymer eine feste Lösung bilden wird. Gerade bei der Entwicklung von Arzneimittel auf der Basis neuer Wirkstoffe stehen jedoch häufig nur kleinere Mengen des Wirkstoffs zur Verfügung, so dass die Möglichkeit einer Vorhersage mit Hilfe eines einfachen Testsystems außerordentlich wünschenswert ist.

Ebenfalls wünschenswert ist die Möglichkeit, hinsichtlich der Stabilität von festen Lösungen oder Feststoffdispersionen Vorhersagen treffen zu können. In Abhängigkeit von der Kompatibilität des Wirkstoffs mit dem Copolymer kann es nämlich zur Entmischung der vorher homogenen dispersen Phase oder zur Rekristallisation des Wirkstoffs kommen. Eine solche Phasenseparation oder Rekristallisation ist wegen der damit verbundenen Änderung der Homogenität und des Freisetzungsverhaltens unerwünscht.

Die EP-A 0987549 offenbart ein Testsystem zur Charakterisierung der Verträglichkeit von biologisch aktiven Substanzen mit Polyvinylpyrrolidon in einer Feststoffdispersion.

Der vorliegenden Erfindung liegt die Aufgabe zugrunde, ein Testsystem anzugeben, mit dessen Hilfe die Kompatibilität von biologisch aktiven Substanzen und N-Vinylpyrrolidon-Copolymeren auf einfache Weise vorhergesagt werden kann.

Überraschenderweise wurde gefunden, dass die Lösungseigenschaften von N-Vinylpyrrolidon-Copolymeren durch ein flüssiges Gemisch von 1,3-Bis-(pyrrolidon-1-yl)-butan mit bestimmten Verbindungen simuliert werden können, die strukturelle Ähnlichkeit mit den im Copolymer vorhandenen Comonomereinheiten aufweisen.

Die Erfindung betrifft daher ein flüssiges Gemisch, das
a) 1,3-Bis-(pyrrolidon-1-yl)-butan und
b) wenigstens eine Verbindung der Formel I 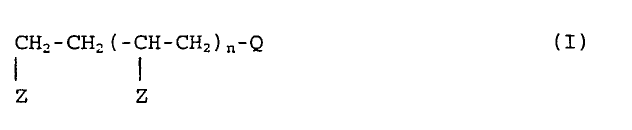
enthält, worin
Q für CH₂-Z, CH₂-CH₂-Z oder CHZ-C₁-C₄-Alkyl steht,
n für 0, 1, 2 oder 3 steht, und
die Reste Z für C₁-C₂₀-Alkylcarbonyloxy, Carboxyl, C₁-C₂₀-Alkyloxy-carbonyl, C₂-C₄-Hydroxyalkyloxycarbonyl, Di(C₁-C₄-alkyl)amino-C₂-C₄₋alkyloxycarbonyl oder Tri(C₁-C₄-alkyl)ammonium-C₂-C₄-alkyloxycarbonyl stehen.

In der Formel I sind vorzugsweise alle vorkommenden Reste Z identisch. "Flüssiges Gemisch" soll für die Zwecke der vorliegenden Anmeldung bedeuten, dass das Gemisch zumindest bei leicht erhöhter Temperatur, z.B. bei 45 °C, vorzugsweise bereits bei Raumtemperatur flüssig vorliegt.

Das flüssige Gemisch dient als Testlösungsmittel, das die Lösungseigenschaften des N-Vinylpyrrolidon-Copolymers simuliert. Das flüssige Gemisch enthält die Komponenten a) und b) üblicherweise in einem Gewichtsverhältnis von 10 : 1 bis 1 : 10, vorzugsweise 5 : 1 bis 1 : 5.

Die Erfindung betrifft außerdem ein Testsystem zur Evaluierung der Kompatibilität einer biologisch aktiven Substanz mit einem Copolymer, das Einheiten des N-Vinylpyrrolidons und wenigstens eines ethylenischen ungesättigten Momomers der Formel II umfasst

CH₂=CR-Z' (II)

worin R für Wasserstoff oder Methyl steht und Z' die oben für Z angegebene Bedeutung hat, wobei das Testsystem das vorstehend definierte flüssige Gemisch und wenigstens eine biologisch aktive Substanz umfasst.

Die Erfindung betrifft außerdem ein Verfahren zur Evaluierung der Kompatibilität einer biologisch aktiven Substanz mit einem N-Vinylpyrrolidon-Copolymer, wobei das Copolymer Einheiten des N-Vinylpyrrolidons in einem Gewichtsanteil xᵥₚ und Einheiten wenigstens eines ethylenisch ungesättigten Monomers der Formel II

CH₂=CR-Z' (II)

worin R für Wasserstoff oder Methyl steht, Z' für C₁-C₂₀-Alkylcarbonyloxy, Carboxyl, C₁-C₂₀-Alkyloxycarbonyl, C₂-C₄-Hydroxyalkyl-oxycarbonyl, Di(C₁-C₄-alkyl)amino-C₂-C₄-alkyloxycarbonyl oder Tri(C₁₋C₄-alkyl)ammonium-C₂-C₄-alkyloxycarbonyl stehen, in einem Gewichtsanteil x_{M} umfasst, bei dem man
a) ein Testlösungsmittel zubereitet, das 1,3-Bis-(pyrrolidon-1-yl)-butan in einem Gewichtsanteil xᵥₚ und eine Verbindung der Formel I in einem Gewichtsanteil x_{M} enthält, 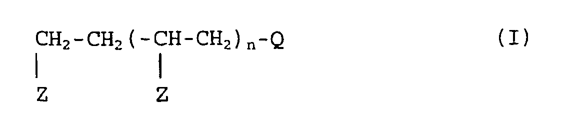 worin Q für CH₂-Z, CH₂-CH₂-Z oder CHZ-C₁-C₄-Alkyl steht, n für 0, 1, 2 oder 3 steht, die Reste Z identisch sind und dem Rest Z' entsprechen,
b) die biologisch aktive Substanz mit dem Testlösungsmittel in Kontakt bringt, und
c) das Phasenverhalten des Gemisches und/oder die Löslichkeit der biologisch aktiven Substanz in dem Testlösungsmittel bestimmt.

x_{VP} beträgt im Allgemeinen 10 bis 90 Gew.-%, meist 30 bis 70 Gew.-%. x_{M} beträgt im Allgemeinen 90 bis 10 Gew.-%, meist 70 bis 30 Gew.-%. Ist mehr als ein Monomer der Formel II vorhanden, so sind für x_{M} die individuellen Beiträge der unterschiedlichen Monomere x_{M1}, x_{M2}, ...zu setzen.

Die Reste Z bzw. Z' stehen vorzugsweise für C₁-C₄-Alkylcarbonyloxy, Carboxyl, C₁-C₄-Alkyloxycarbonyl oder C₂-C₄-Hydroxyalkyloxycarbonyl. Wenn Z oder Z' für Tri(C₁-C₄-alkyl)ammonium-C₂-C₄-alkyloxycarbonyl stehen, sind sie von einem Äquivalent eines pharmazeutisch akzeptablen Anions begleitet, wie Hydroxid, Sulfat, Hydrogensulfat, Carbonat, Hydrogencarbonat, einem Halogenid, insbesondere Chlorid, dem Anion einer organischen Säure wie Acetat, Lactat, Fumarat, oder dergleichen. Wenn Z oder Z' für Carboxyl stehen, kann die Carboxylgruppe auch ganz oder teilweise neutralisiert sein, wobei als ladungsausgleichende Kationen pharmazeutisch akzeptable Kationen wie Alkalimetall- oder Erdalkalimetallionen, z.B. Natrium oder Kalium, oder unsubstituierte oder substituierte Ammoniumionen, wie Dimethylammonium, Trimethylammonium, Diethanolammonium und dergleichen, in Betracht kommen.

1,3-Bis-(pyrolidon-1-yl)-butan kann durch Dimerisierung von N-Vinylpyrrolidon unter sauren Reaktionsbedingungen und nachfolgende Hydrierung des erhaltenen 1,3-Bis-(pyrrolidon-1-yl)-butens zum 1,3-Bis-(pyrrolidon-1-yl)-butan erhalten werden (vgl. Breitenbach et al., Naturwissenschaften 42, 955,155; 440). 1,3-Bis-(pyrrolidon-1-yl)-butan stellt eine ölige, farblose Flüssigkeit mit einem Siedepunkt von 205 bis 215° C (0,2 mbar) dar.

Die Verbindungen der allgemeinen Formel I sind entweder handelsüblich oder können auf einfache Weise hergestellt werden. Mit Erfolg wurden beispielsweise 1,3-Diacetoxybutan und insbesondere 1,4-Diacetoxybutan als Verbindung der Formel I eingesetzt. Verbindungen der Formel I können z.B. durch Veresterung von Polyolen wie 1,3-Butandiol, 1,4-Butandiol oder 1,3,5-Pentantriol mit Carbonsäuren wie Essigsäure oder deren Derivaten oder durch Veresterung von Polycarbonsäuren wie Glutarsäure oder Adipinsäure mit geeigneten Alkoholen erhalten werden.

Als Copolymere, deren Kompatibilität mit biologisch aktiven Substanzen mit Hilfe des erfindungsgemäßen Testsystems evaluiert werden kann, kommen Copolymere des N-Vinylpyrrolidon mit ethylenisch ungesättigten Monomeren der Formel II

CH₂=CR-Z' (II)

in Betracht, worin R für Wasserstoff oder Methyl steht und Z' für C₁₋C₂₀-Alkylcarbonyloxy, Carboxyl, C₁-C₂₀-Alkyloxycarbonyl, C₂-C₄₋Hydroxyalkyloxycarbonyl, Di(C₁-C₄-alkyl)amino-C₂-C₄-alkyloxycarbonyl oder Tri(C₁-C₄-alkyl)ammonium-C₂-C₄-alkyloxycarbonyl steht.

Als Monomere der Formel II lassen sich Vinylester von C₁-C₂₀-Alkancarbonsäuren, wie Vinylacetat oder Vinylpropionat, Acryl- oder Methacrylsäure, C₁-C₂₀-Alkylester der Acrylsäure oder Methacrylsäure, wie Methylacrylat, Methylmethacrylat, Ethylacrylat, Ethylmethacrylat, C₂₋C₄-Hydroxyalkyl(meth)acrylate, wie Hydroxyethylacrylat, Di(C₁-C₄₋alkyl)amino-C₂-C₄-alkyl(meth)acrylate, wie Dimethylaminopropylacrylat, oder (Meth)acryloyloxy-C₂-C₄-alkyl -tri(C₁-C₄-alkyl)ammoniumsalze, wie Acryloyloxypropyl-trimethylammonium-chlorid, anführen.

Zu den bevorzugten Copolymeren zählen solche von N-Vinylpyrrolidon und Vinylacetat, insbesondere in einem Gewichtsverhältnis von 70 : 30 bis 30 : 70; und Copolymere des N-Vinylpyrrolidons mit Methylmethacrylat, insbesondere in einem Gewichtsverhältnis von 20 : 80 bis 55 : 45.

Die Copolymere weisen im Allgemeinen einen K-Wert nach Fikentscher von 10 bis 110, insbesondere von 20 bis 80 auf.

Der Rest Z in der Verbindung der Formel I wird entsprechend dem Rest Z' im Comonomer des zu simulierenden Copolymers gewählt. So dient ein Gemisch von 1,3-Bis-(pyrrolidon-1-yl)-butan mit 1,4-Diacetoxybutan beispielsweise zur Simulation der Lösungseigenschaften von N-Vinylpyrrolidon/Vinylacetat-Copolymeren. Selbstverständlich kann das zu simulierende Copolymer auch zwei oder mehrere unterschiedliche Monomere der Formel II enthalten. Zur Zubereitung des Testlösungsmittels werden dann zwei oder mehrere unterschiedliche Verbindungen der Formel I mit entsprechend gewählten Resten Z als Komponente b) eingesetzt.

Kompatibilität beziehungsweise Verträglichkeit im Sinne der vorliegenden Anmeldung bezeichnet die Fähigkeit einer Substanz, mit dem N-Vinylpyrrolidon-Copolymer eine homogene, stabile Feststoffdispersion auszubilden, wobei diese Feststoffdispersion insbesondere eine feste Lösung, das heißt eine molekulardisperse Verteilung der Komponenten ineinander, darstellt. Das Testsystem eignet sich prinzipiell für alle pharmazeutischen Wirkstoffe, Pflanzenschutzmittel, Nahrungsergänzungsmittel oder kosmetische Wirkstoffe. Weiterhin lassen sich auch Waschmittel oder Farbstoffe auf ihre Verträglichkeit mit den Copolymeren untersuchen. Auch der Einfluss von Formulierungshilfsmitteln, die selbst nicht biologisch aktiv sind, wie Zucker, Zuckeralkoholen, Solubilisatoren, wie Tensiden, oder weiteren polymeren Hilfsmittel, kann untersucht werden.

Zur Durchführung des erfindungsgemäßen Verfahrens wird zunächst ein Testlösungsmittel zubereitet. Das Testlösungsmittel enthält 1,3-Bis-(pyrrolidon-1-yl)-butan und eine Verbindung der Formel I in einem Gewichtsverhältnis, das dem von N-Vinylpyrrolidon und Comonomer(en) im zu simulierenden Copolymer entspricht. Es wird dann die Löslichkeit der zu untersuchenden biologisch aktiven Substanz bei einer bestimmten Temperatur, meist Raumtemperatur, in dem flüssigen Gemisch beurteilt. Man kann die Löslichkeit quantitativ bestimmen, z.B. in Gew.-%, bezogen auf das Gewicht von Testlösungsmittel und biologisch aktiver Substanz. In vielen Fällen ist eine Aussage darüber ausreichend, ob die Löslichkeit größer oder kleiner ist als ein bestimmter Wert. Hierzu wird eine vorbestimmte Menge der biologisch aktiven Substanz mit dem Testlösungsmittel in Kontakt gebracht. Die Mengenverhältnisse sind im Prinzip frei wählbar. Es empfiehlt sich jedoch, die Konzentrationsbereiche in dem Testsystem so zu wählen, dass sie den für Extrudatformen typischen Wirkstoffgehalt entsprechen, also im Allgemeinen 0,1 bis 70 Gew.-% bevorzugt 10 bis 30 Gew.-% an biologische aktiver Substanz, bezogen auf das Gesamtgewicht des Testsystems.

Die biologisch aktive Substanz wird üblicherweise eingewogen, mit dem Testlösungsmittel versetzt und vorzugsweise durchmischt, z.B. mit einem Labormagnetrührer bei 5 bis 2000 Upm gerührt oder mit Ultraschall oder einem Vortex-Homogenisator behandelt. Zur Beschleunigung der Auflösung kann man das Testsystem auch erwärmen. Bevorzugt erfolgt das Erwärmen so, dass es in etwa der Aufheizrate in einer Schmelzformulierung entspricht, also mit 0,5 bis 5° C/min. Vorzugsweise wird das Testsystem auf maximal 140° C, z.B. auf eine Temperatur im Bereich von 45 bis 140 °C oder 110 bis 140 °C, erwärmt. Im Einzelfall kann man aber auch bis zum Siedepunkt des flüssigen Gemisches erwärmen. Anschließend lässt man das Testsystem auf die Bestimmungstemperatur, meist Raumtemperatur, abkühlen.

Man beurteilt dann das Phasenverhalten des Gemisches, d.h. durch visuelle, spektroskopische und/oder thermoanalytische Untersuchung der erhaltenen Mischung stellt man fest, ob die biologisch aktive Substanz mit dem flüssigen Gemisch eine einheitliche Phase zu bilden vermag.

Die visuelle Betrachtung erfolgt beispielsweise mit einem Mikroskop, wie einem üblichen Lichtmikroskop. Dabei wird festgestellt, ob sich eine klare Lösung gebildet hat. Neben der visuellen Betrachtung eignet sich auch eine spektroskopische Untersuchung des Testsystems. Z.B. kann man das Testsystem mit Hilfe der konfokalen Ramanspektroskospie auf seinen amorphen Charakter untersuchen. Außerdem eignet sich die Methode der Differentialscanningkalorimetrie. Aus dem Vorliegen einer einheitlichen Phase lässt sich schließen, dass die Löslichkeit der biologisch aktiven Substanz größer ist, als die Konzentration der Substanz im Lösungsversuch. Umgekehrt kann aus dem Auftreten einer Phasenseparation auf eine geringere Löslichkeit geschlossen werden.

Zur quantitativen Bestimmung der Löslichkeit kann man z.B. wie folgt vorgehen:
a) Es wird eine Konzentrationsreihe hergestellt, indem man in Parallelversuchen verschiedene Mengen an biologisch aktiver Substanz mit einer konstanten Menge Testlösungsmittel in Kontakt bringt. Nach einer Äquilibrationszeit über eine definierte Zeitspanne bei einer gegebenen Temperatur, vorzugsweise unter Durchmischung, z.B. 24 Stunden unter Rühren oder 30 min unter Ultraschallbehandlung, wird ermittelt, bis zu welcher maximalen Konzentration klare Lösungen erhalten werden. Die Löslichkeit der biologisch aktiven Substanz liegt zwischen den Konzentrationen, für die noch eine klare Lösung bzw. keine klare Lösung mehr erhalten wird.
b) Man versetzt eine Menge der biologisch aktiven Substanz mit einer zur vollständigen Auflösung nicht ausreichenden Menge des Testlösungsmittels bzw. gibt solange weitere Mengen an biologisch aktiver Substanz zur Lösung, bis sich die zugegebene Menge nicht mehr vollständig löst. Nach einer Äquilibrationszeit über eine definierte Zeitspanne bei einer gegebenen Temperatur, vorzugsweise unter Durchmischung, z.B. 24 Stunden unter Rühren oder 30 min unter Ultraschallbehandlung, wird eine Probe des klaren Überstands entnommen. Hierzu kann man den Ansatz vorher zentrifugieren, z.B. mit Hilfe einer Ultrazentrifuge bei 8000 bis 12000 Upm. In der Probe des klaren Überstands wird die Konzentration der biologisch aktiven Substanz z.B. durch Hochdruckflüssigkeitschromatographie (HPLC) bestimmt. Der gefundene Wert entspricht der Löslichkeit der biologisch aktiven Substanz.

Anhand der vorstehend genannten Methoden kann abhängig von den Äquilibrationsbedingungen die thermodynamische Sättigungslöslichkeit oder maximale (kinetische) Löslichkeit bestimmt werden.

Die nach 24 Stunden bei Raumtemperatur (22 °C +/- 2 °C) ermittelte Löslichkeit entspricht im Wesentlichen der thermodynamischen Sättigungslöslichkeit der biologisch aktiven Substanz. Dieser Löslichkeitswert ist ein Maß für die thermodynamische Sättigungslöslichkeit der biologisch aktiven Substanz in der Matrix des Copolymers bei Raumtemperatur. Feste Lösungen von biologisch aktiven Substanzen sind thermodynamisch stabil, wenn die Wirkstoffbeladung unterhalb der thermodynamischen Sättigungslöslichkeit der biologisch aktiven Substanz in der Matrix liegt.

Durch Energieeintrag kann die Wirkstoffbeladung in festen Lösungen jedoch stark erhöht werden. Die maximal erreichbare Wirkstoffbeladung einer gegebenen Matrix eines Copolymer kann mit dem erfindungsgemäßem Testsystem vorhergesagt werden, indem man die Löslichkeit der biologisch aktiven Substanz gemäß einer der vorstehenden Methoden a) oder b) bestimmt, wobei man die Äquilibration unter Erwärmen auf eine Temperatur von z.B. bis zu 140° C, insbesondere 110 bis 140° C, oder unter Ultrabeschallung durchführt.

Das erfindungsgemäße Testsystem gestattet auch die Vorhersage des Rekristallisationsverhaltens. Vor allem bei Testsystemen, bei denen durch Energieeintrag, z.B. Erwärmen oder Ultrabeschallung, höhere Wirkstoffbeladungen als die thermodynamische Sättigungslöslichkeit eingestellt wurden, stellt das Rekristallisationsverhalten nach Beendigung des Energieeintrags bzw. nach dem Abkühlen auf Raumtemperatur ein wichtiges Kriterium dar. Testsysteme, in denen die biologisch aktive Substanz nicht unmittelbar rekristallisiert, werden auf Langzeitstabilität untersucht. Hierzu können beispielsweise folgende Bedingungen angewandt werden:
- Stehenlassen bei Raumtemperatur über 24 Stunden,
- Lagerung über einen Monat, 3 Monate, 6 Monate in Klimazone 2 (25 °C, 60% relative Luftfeuchte) oder Klimazone 4 (20 °C, 70% relative Luftfeuchte), oder
- Streßlagerung bei 40 °C, 75% relative Luftfeuchte über bis zu 6 Monate.

Mit Hilfe des erfindungsgemäßen Testsystems lässt sich auch der Einfluß von Hilfsstoffen oder eine löslichkeitserhöhende oder löslichkeitserniedrigende Wirkung der Gegenwart einer zweiten oder weiteren biologisch aktiven Substanz auf die Löslichkeit einer ersten biologisch aktiven Substanz in der Matrix des Copolymers untersuchen. Dazu werden diese Hilfsstoffe, z.B. Solubilisatoren beziehungsweise weitere biologisch aktive Substanzen, neben der zu testenden biologisch aktiven Substanz dem Testlösungsmittel zugesetzt. Danach können wiederum die thermodynamische Sättigungslöslichkeit und/oder die maximale Löslichkeit bestimmt werden, wie oben angegeben.

Die Erfindung wird durch die folgenden Beispiele näher veranschaulicht.

### Beispiel 1: Thermodynamische Sättigungslöslichkeit von Lopinavir in einer Matrix eines Copolymers von N-Vinylpyrrolidon und Vinylacetat (60:40)

Das Copolymer wurde durch ein Gemisch von 1,3-Bis(pyrrolidon-1-yl)-butan und 1,4-Diacetoxybutan im Gewichtsverhältnis von 6 : 4 simuliert.

### Methode a)

In Parallelversuchen wurde der Wirkstoff in Glasfläschen eingewogen und mit dem Testlösungsmittel aufgefüllt, entsprechend den in der nachstehenden Tabelle angegebenen Konzentrationen (alle Konzentrationen in Gew./Gew.). Sämtliche sieben Proben wurden mit einem Magnetrührstäbchen versehen und 24 Stunden bei Raumtemperatur gerührt. Durch visuelle Beobachtung stellte man fest, dass der Wirkstoff bis 24,1% klar löslich war; der Versuch mit 26,0% zeigte unvollständige Auflösung. Die Sättigungslöslichkeit lag daher zwischen 24,1 und 26,0 Gew.-%.

| | | | | | | | |
|---|---|---|---|---|---|---|---|
| Lopinavir [%] | 20,2 | 21,9 | 24,1 | 26,0 | 29,5 | 33,9 | 36,3 |
| Klare Lösung | + | + | + | - | - | - | - |

### Methode b)

In einer alternativen Bestimmungsmethode wurden 150 mg Lopinavir mit 350 mg des Testlösungsmittels versetzt und 24 Stunden bei Raumtemperatur gerührt. Anschließend wurde die Probe eine Minute bei 12000 Upm zentrifugiert. Der klare Überstand wurde mittels HPLC untersucht. Man fand eine Löslichkeit von 24,9 Gew.-%.

### Methode c)

Zur Bestimmung der maximalen Löslichkeit wurden 60 mg Lopinavir mit 140 mg des Testlösungsmittels versetzt und 30 min bei Raumtemperatur mit Ultraschall behandelt. Anschließend wurde die Probe 10 min bei 10000U pm zentrifugiert. Eine Probe des klaren Überstands wurde mittels HPLC untersucht. Man fand eine maximale Löslichkeit von 39,72 Gew.-%. Die Restmenge der Probe wurde 4 Wochen bei Raumtemperatur gelagert. Dann wurde eine weitere Probe des Überstands entnommen und per HPLC untersucht. Man fand eine Konzentration von 28,87 Gew.-%.

### Beispiel 2: Thermodynamische Sättigungslöslichkeit von Lopinavir unter Einfluß von Polyoxyethylenglyceroltrihydroxystearat (Cremophor RH 40®)

Beispiel 1a) wurde wiederholt, wobei jedoch die in der nachstehenden Tabelle angegebenen Konzentrationen verwendet wurden und das Testlösungsmittel 5 Gew.-% Cremophor RH 40 enthielt. Man ermittelte eine Sättigungslöslichkeit zwischen 22,0 und 26,0%.

| | | | | | | | |
|---|---|---|---|---|---|---|---|
| Lopinavir [%] | 20,0 | 22,0 | 23,7 | 26,0 | 30,0 | 33,0 | 35,8 |
| Cremophor [%]* | 5 | 5 | 5 | 5 | 5 | 5 | 5 |
| Klare Lösung | + | + | + | - | - | - | - |

| | | | | | | | |
|---|---|---|---|---|---|---|---|
| * bezogen auf das Testlösungsmittel | | | | | | | |

### Beispiel 1b) wurde wiederholt, wobei jedoch das Testlösungsmittel 5 Grew.-% Cremophor RH 40 enthielt. Es wurde eine Löslichkeit von 22,8% ermittelt.

Mit Hilfe des erfindungsgemäßen Testsystems konnte gezeigt werden, dass Cremophor RH 40 die thermodynamsiche Sättigungslöslichkeit von Lopinavir senkt.

### Beispiel 3: Thermodynamische Sättigungslöslichkeit von Lopinavir unter Einfluß von Ritonavir

Es wurde eine Wirkstoffvormischung der beiden oben genannten Wirkstoffe in ein Gewichtsverhältnis von 4 : 1 hergestellt. Mit dieser Wirkstoffmischung wurde Beispiel 1a) wiederholt, wobei die Konzentration wie in der nachstehenden Tabelle waren.

| | | | | |
|---|---|---|---|---|
| Lopinavir [%] | 19,2 | 20,6 | 23,9 | 27,8 |
| Ritonavir [%] | 4,8 | 5,2 | 6 | 6,9 |
| Wirkstoff ges. [%] | 24,0 | 25,8 | 29,9 | 34,7 |
| Klare Lösung | + | + | - | - |

Beispiel 1b) wurde mit der vorstehend genannten Wirkstoffvormischung wiederholt. Im klaren Überstand wurden mittels HPLC 21,3 Gew.-% Lopinavir ermittelt. Mit Hilfe des erfindungsgemäßen Testsystems konnte gezeigt werden, dass die Gegenwart von Ritonavir die thermodynamische Sättigungslöslichkeit von Lopinavir senkt.

### Beispiel 4: Thermodynamische Sättigungslöslichkeit von Lopinavir unter Einfluß von Ritonavir und Cremophor RH 40

Beispiel 3a) wurde wiederholt, wobei die in der nachstehenden Tabelle angegebenen Konzentrationen verwendet und das Testlösungsmittel 10 Gew.-% Cremophor RH 40 enthielt. Die visuelle Bestimmung der Konzentrationsreihe führte zu einer thermodynamischen Sättigungslöslichkeit der Wirkstoffmischung zwischen 23,6% und 28,0%. Die HPLC-Methode führte zu einem Ergebnis von 21,7% für Lopinavir.

| | | | | |
|---|---|---|---|---|
| Lopinavir [%] | 14,2 | 15,8 | 18,9 | 22,4 |
| Ritonavir [%] | 3,6 | 4,0 | 4,7 | 5,6 |
| Wirkstoff ges. [%] | 17,8 | 19,8 | 23,6 | 28,0 |
| Cremophor [%] * | 10 | 10 | 10 | 10 |
| Klare Lösung | + | + | + | - |

| | | | | |
|---|---|---|---|---|
| * bezogen auf das Testlösungsmittel | | | | |

### Beispiel 5: Schmelzextrusion

Es wurde eine Formulierung für die Schmelzextrusion hergestellt, die 18,7 Gew.-Teile Lopinavir, 4,7 Gew.-Teile Ritonavir, 10,0 Gew.-Teile Cremophor RH 40 und 100 Gew.-Teile N-Vinylpyrrolidon/Vinylacetat-Copolymer (60:40) enthielt. Bei der Schmelzextrusion dieser Formulierung mittels eines beheizten Doppelschneckenextruders wurden stabile feste Lösungen erhalten. Nach 8 Monaten Lagerung bei Raumtemperatur wurden die Extrudate röntgenographisch untersucht. Beide Wirkstoffe lagen röntgenamorph vor, d.h. es fand keine Rekristallisation eines Wirkstoff statt. Entsprechend der Vorhersage des Testsystem liegt eine stabile feste Lösung der Wirkstoffe vor.

## Patentansprüche

1. Flüssiges Gemisch, enthaltend
a) 1,3-Bis-(pyrrolidon-1-yl)-butan und
b) wenigstens eine Verbindung der Formel I 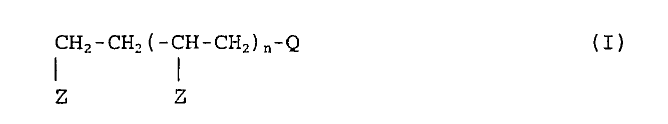
worin
Q für CH₂-Z, CH₂-CH₂-Z oder CHZ-C₁-C₄-Alkyl steht,
n für 0, 1, 2 oder 3 steht, und
die Reste Z für C₁-C₂₀-Alkylcarbonyloxy, Carboxyl, C₁-C₂₀-Alkyl-oxycarbonyl, C₂-C₄-Hydroxyalkyloxycarbonyl, Di(C₁-C₄-alkyl)amino-C₂-C₄-alkyloxycarbonyl oder Tri (C₁-C₄-alkyl)ammonium-C₂-C₄₋alkyloxycarbonyl stehen.

2. Gemisch nach Anspruch 1, wobei es sich bei der Komponente b) um 1,4-Diacetoxybutan handelt.

3. Gemisch nach Anspruch 1 oder 2, worin die Komponenten a) und b) in einem Gewichtsverhältnis von 10:1 bis 1:10 vorliegen.

4. Testsystemsystem zur Evaluierung der Kompatibilität einer biologisch aktiven Substanz mit einem Copolymer, das Einheiten von N-Vinylpyrrolidon und wenigstens eines ethylenischen ungesättigten Momomers der Formel II umfasst
CH₂=CR-Z' (II)
worin R für Wasserstoff oder Methyl steht und Z' die in Anspruch 1 für Z angegebene Bedeutung hat, wobei das Testsystem ein flüssiges Gemisch nach einem der Ansprüche 1 bis 3 und wenigstens eine biologisch aktive Substanz umfasst.

5. Testsystem nach Anspruch 4, enthaltend 10 bis 70 Gew.-% an biologisch aktiver Substanz.

6. Testsystem nach Anspruch 4 oder 5, außerdem enthaltend wenigstens ein Formulierungshilfsmittel.

7. Verfahren zur Evaluierung der Kompatibilität einer biologisch aktiven Substanz mit einem N-Vinylpyrrolidon-Copolymer, das Einheiten des N-Vinylpyrrolidons in einem Gewichtsanteil xᵥₚ und Einheiten wenigstens eines ethylenisch ungesättigten Monomers der Formel II
CH₂=CR-Z' (II)
worin R für Wasserstoff oder Methyl steht, Z' für C₁-C₂₀-Alkylcarbonyloxy, Carboxyl, C₁-C₂₀-Alkyloxycarbonyl, C₂-C₄-Hydroxy-alkyloxycarbonyl, Di(C₁-C₄-alkyl)amino-C₂-C₄-alkyloxycarbonyl oder Tri(C₁-C₄-alkyl)ammonium-C₂-C₄-alkyloxycarbonyl stehen, in einem Gewichtsanteil x_{M} umfasst, bei dem man
a) ein Testlösungsmittel zubereitet, das 1,3-Bis-(pyrrolidon-1-yl)-butan in einem Gewichtsanteil xᵥₚ und eine Verbindung der Formel I in einem Gewichtsanteil x_{M} enthält, 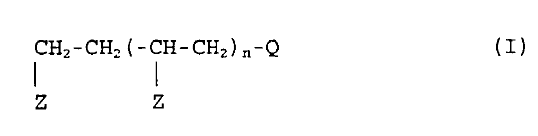 worin Q für CH₂-Z, CH₂-CH₂-Z oder CHZ-C₁-C₄-Alkyl steht, n für 0, 1, 2 oder 3 steht, die Reste Z identisch sind und dem Rest Z' entsprechen,
b) die biologisch aktive Substanz mit dem Testlösungsmittel in Kontakt bringt, und
c) das Phasenverhalten des Gemisches und/oder die Löslichkeit der biologisch aktiven Substanz in dem Testlösungsmittel bestimmt.

8. Verfahren nach Anspruch 7 bei dem man das Phasenverhalten des Gemisches visuell, spektroskopisch und/oder thermoanalytisch untersucht.

9. Verfahren nach Anspruch 7 oder 8, bei dem man das Gemisch aus biologisch aktiver Substanz und Testlösungsmittel auf eine Temperatur von bis zu 140° C erwärmt.

## Claims

1. Liquid mixture containing
a) 1,3-bis-(pyrrolidon-1-yl)-butane and
b) at least one compound of formula I 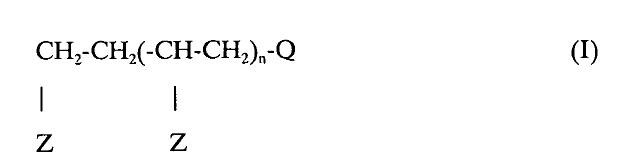
wherein
Q denotes CH₂-Z, CH₂-CH₂-Z or CHZ-C₁₋₄-alkyl,
n denotes 0, 1, 2 or 3, and
the groups Z denote C₁-C₂₀-alkylcarbonyloxy, carboxyl, C₁-C₂₀-alkyloxycarbonyl, C₂-C₄-hydroxyalkyloxycarbonyl, di(C₁₋₄-alkyl)amino-C₂-C₄-alkyloxycarbonyl or tri(C₁₋₄-alkyl)ammonium-C₂-C₄-alkyloxycarbonyl.

2. Mixture according to claim 1, wherein component b) is 1,4-diacetoxybutane.

3. Mixture according to claim 1 or 2, wherein components a) and b) are present in a weight ratio of 10:1 to 1:10.

4. Test system for evaluating the compatibility of a biologically active substance with a copolymer which contains units of N-vinylpyrrolidone and at least one ethylenically unsaturated monomer of formula II
CH₂=CR-Z' (II)
wherein R denotes hydrogen or methyl and Z' has the meaning given for Z in claim 1, the test system comprising a liquid mixture according to one of claims 1 to 3 and at least one biologically active substance.

5. Test system according to claim 4, containing 10 to 70 wt.% of biologically active substance.

6. Test system according to claim 4 or 5, also containing at least one formulation adjuvant.

7. Method of evaluating the compatibility of a biologically active substance with an N-vinylpyrrolidone copolymer which contains units of the N-vinylpyrrolidone in an amount by weight of xᵥₚ and units of at least one ethylenically unsaturated monomer of formula II
CH₂=CR-Z' (II)
wherein R denotes hydrogen or methyl, Z' denotes C₁-C₂₀-alkylcarbonyloxy, carboxyl, C₁-C₂₀-alkyloxycarbonyl, C₂-C₄-hydroxyalkyloxycarbonyl, di(C₁₋₄₋alkyl)amino- C₂-C₄-alkyloxycarbonyl or tri(C₁₋₄-alkyl)ammonium-C₂-C₄₋alkyloxycarbonyl, in an amount by weight of x_{M}, wherein
a) a test solvent is prepared which contains 1,3-bis-(pyrrolidon-1-yl)-butane in an amount by weight of xᵥₚ and a compound of formula I in an amount by weight of x_{M}, 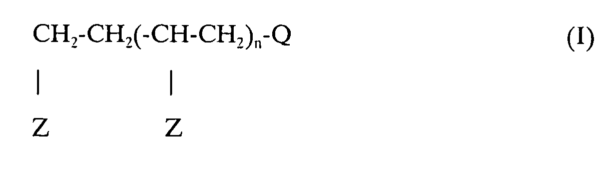 wherein Q denotes CH₂-Z, CH₂-CH₂-Z or CHZ-C₁₋₄-alkyl, n denotes 0, 1, 2 or 3, the groups Z are identical and correspond to the group Z',
b) the biologically active substance is brought into contact with the test solvent, and
c) the phase characteristics of the mixture and/or the solubility of the biologically active substance in the test solvent is/are determined.

8. Method according to claim 7, wherein the phase characteristics of the mixture are investigated visually, spectroscopically and/or thermoanalytically.

9. Method according to claim 7 or 8, wherein the mixture of biologically active substance and test solvent is heated to a temperature of up to 140°C.

## Revendications

1. Mélange liquide contenant
a) du 1,3-bis-(pyrrolidone-1-yl)-butane et
b) au moins un composé de formule I 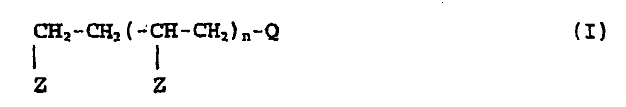
dans laquelle
Q représente un groupe CH₂-Z, CH₂-CH₂-Z ou CHZ-alkyle en C₁ à C₄,
n représente 0, 1, 2 ou 3 et
les radicaux Z représentent l'alkylcarbonyloxy en C₁ à C₂₀, carboxyle, alkyloxycarbonyle en C₁ à C₂₀, hydroxyalkyloxycarbonyle en C₂ à C₄, di(alkyl en C₁ à C₄)amino-alkyloxycarbonyle en C₂ à C₄ ou tri (alkyl en C₁ à C₄)ammonium-alkyloxycarbonyle en C₂ à C₄.

2. Mélange selon la revendication 1, dans lequel le composant b) est le 1,4-diacétoxybutane.

3. Mélange selon la revendication 1 ou 2, dans lequel les composants a) et b) sont présents dans un rapport pondéral de 10:1 à 1:10.

4. Système de test pour l'évaluation de la compatibilité d'une substance biologiquement active avec un copolymère qui comprend des unités N-vinylpyrrolidone et au moins un monomère à insaturation éthylénique de formule II
CH₂=CR-Z' (II)
dans laquelle R représente de l'hydrogène ou un radical méthyle et Z' a la signification indiquée pour Z dans la revendication 1, le système de test comprenant un mélange liquide selon l'une quelconque des revendications 1 à 3 et au moins une substance biologiquement active.

5. Système de test selon la revendication 4, comprenant 10 à 70 % en poids de substance biologiquement active.

6. Système de test selon la revendication 4 ou 5, comprenant en outre au moins un adjuvant de formulation.

7. Procédé d'évaluation de la compatibilité d'une substance biologiquement active comportant un copolymère de N-vinylpyrrolidone qui comprend, dans une proportion pondérale xᵥₚ, des unités N-vinylpyrrolidone et des unités d'au moins un monomère à insaturation éthylénique de formule II
**CH**_{**2**}**=CR-Z'** (II)
dans laquelle R représente de l'hydrogène ou un radical méthyle, Z' un radical alkylcarbonyloxy en C₁ à C₂₀, carboxyle, alkyloxycarbonyle en C₁ à C₂₀, hydroxyalkyloxycarbonyle en C₂ à C₄, di(alkyl en C₁ à C₄)amino-alkyloxycarbonyle en C₂ à C₄ ou tri(alkyl en C₁ à C₄)ammonium-alkyloxycarbonyle en C₂ à C₄, dans une proportion pondérale x_{M}, procédé dans lequel
a) on prépare un solvant d'essai qui comprend du 1,3-bis(pyrrolidone-1-yl)-butane dans une proportion pondérale xᵥₚ et un composé de formule I dans une proportion pondérale x_{M} 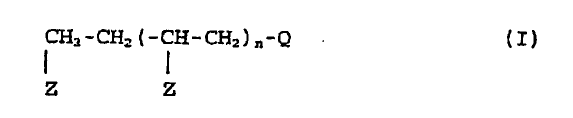 dans laquelle Q représente un groupe CH₂-Z, CH₂-CH₂-Z ou CHZ-alkyle en C₁ à C₄, n représente 0, 1, 2 ou 3, les radicaux Z sont identiques et correspondent au radical Z',
b) on met en contact la substance biologiquement active avec le solvant d'essai et
c) on détermine le comportement des phases du mélange et/ou la solubilité de la substance biologiquement active dans le solvant d'essai.

8. Procédé selon la revendication 7 dans lequel on étudie visuellement le comportement des phases du mélange, par spectroscopie et/ou par thermoanalyse.

9. Procédé selon la revendication 7 ou 8, dans lequel on réchauffe le mélange constitué de substance biologiquement active et le solvant d'essai à une température pouvant atteindre 140°C.
